# NEUE EUROPÄISCHE PATENTSCHRIFT

(11) **EP 1 633 414 B2**
(45) Veröffentlichungstag und Bekanntmachung des Hinweises auf die Entscheidung über den Einspruch: **17.10.2012**
(45) Hinweis auf die Patenterteilung: 23.04.2008
(21) Anmeldenummer: 04739948.0
(22) Anmeldetag: 16.06.2004
(51) Int. Cl.: A61M 5/145, A61M 5/142

(54) **MODULARE INFUSIONSPUMPE**
MODULAR INFUSION PUMP
POMPE DE PERFUSION MODULAIRE

(30) Priorität: 17.06.2003 DE 10327254
(43) Veröffentlichungstag der Anmeldung: 15.03.2006
(73) Patentinhaber: F. Hoffmann-La Roche AG, 4070 Basel (CH); Roche Diagnostics GmbH, 68305 Mannheim (DE)
(72) Erfinder: REMDE, Axel, CH-3432 Luetzelflueh (CH); SCHILTGES, Gilbert, CH-3422 Kirchberg (CH)
(74) Vertreter: Schwabe - Sandmair - Marx
(86) Internationale Anmeldenummer: PCT/EP2004/006482
(87) Internationale Veröffentlichungsnummer: WO 2004/110526

(56) Entgegenhaltungen:
- EP-A1- 1 177 802
- EP-B1- 0 976 020
- WO-A1-00/19887
- WO-A1-01/70307
- DE-A1- 19 605 827
- US-A- 0 072 733
- US-A- 0 073 952
- US-A- 5 062 834
- US-A- 5 510 690
- US-A- 5 984 894
- US-A- 6 074 369
- US-A- 6 165 155
- US-A1- 2002 169 439
- US-B1- 6 200 293
- ENERGIZER-ER-DVD Universal Battery/Charger - Anzeige Amazon

## Beschreibung

Die Erfindung betrifft eine modulare Infusionspumpe für die Verabreichung eines Produkts, vorzugsweise von Insulin oder eines anderen Medikaments.

Insbesondere in der Selbstverabreichung von Medikamenten, beispielsweise Insulin, legen die Verwender, die sich das betreffende Medikament mittels Infusionspumpe selbst verabreichen, zunehmend Wert auf Bequemlichkeit und Diskretion. Die Hersteller kommen den Forderungen unter anderem durch Aufteilung der Infusionspumpe in Baugruppen entgegen, die je in einem eigenen Gehäuse angeordnet und drahtlos oder leitungsgebunden miteinander verbunden sind.

Solch modulare Infusionspumpen sind beispielsweise aus der DE 30 35 670 A1 und der DE 198 40 965 A1 bekannt. Danach wird eine Infusionspumpe in einen unmittelbar am Körper oder sogar im Körper implantierten Pumpenkopf, der ein das Produkt enthaltendes Reservoir und eine Fördereinrichtung umfasst, und ein Bedien- und Steuerteil aufgespalten, das in einem Abstand von dem Pumpenkopf beispielsweise auf oder in der Kleidung getragen werden kann. Das Bedien- und Steuerteil und der Pumpenkopf kommunizieren drahtlos miteinander.

Aus der US 6,200,293 B1 ist eine Infusionspumpe gemäß dem Oberbegriff des Anspruchs bekannt, die ein Fördergerät 185 und ein separates Steuergerät 202 mit einer Energiequelle 222 umfasst, wobei die beiden Geräte über ein elektrisches Kabel 206 verbunden sind. Das Fördergerät 185 weist in einer ersten Ausführungsform ein Reservoir 44, einen Aktuator 50 und eine Kanüle 77 auf. Das Fördergerät kann unter der Kleidung auf der Haut des Patienten platziert werden. In anderen Ausführungen wird die Verbindung zwischen dem Reservoir 244 und einem Kathederkopf 165 über einen Katheder 254 hergestellt. Der Katheder 254 ist dabei mit dem das Reservoir 244 umfassenden Basisgehäuse 242 lösbar über eine Schnellkupplung 250 verbunden. Die US 2002/0169439 A1 beschreibt eine modulare Infusionsvorrichtung und Methode. Die Vorrichtung 10 zur Abgabe des Fluids besteht dabei aus wenigstens zwei Teilen, einem wieder benutzbaren Teil 700 und einem Wegwerfteil 800. Die beiden Teile 700, 800 müssen zum Einsatz der Vorrichtung 10 miteinander verbunden, das heißt, zusammengebaut werden. Ein elektronisch kontrolliertes Vielweg-Injektionssystem zeigt die US 6,165,155 A, bestehend aus einem Gehäuse 250, in das ein mit einem Fluid gefüllter Behälter 300, 506 eingesetzt werden kann, Der Behälter weist auf der distalen Seite einen Ausgang für das Fluid auf, beispielsweise eine Düse 350 oder Nadel 502, und ist auf der proximalen Seite mit einem Kolben 320 verschlossen. Auf den Kolben kann eine Kraft ausgewirkt werden, die diesen in die distale Richtung treibt und so die Ausschüttung des Fluids aus dem Behälter 300, 506 bewirkt.

Es ist eine Aufgabe der Erfindung, den Tragekomfort solch modularer Infusionspumpen weiter zu verbessern.

Die Erfindung betrifft demgemäß eine modulare Infusionspumpe für die Verabreichung eines Produkts, die ein Pumpengehäuse, ein Reservoir für das Produkt, eine

Fördereinrichtung und eine Energiequelle umfasst. Das Pumpengehäuse ist so ausgebildet, dass es mit einer Unterseite unmittelbar auf menschlicher oder tierischer Haut angebracht werden kann. Das Reservoir kann unmittelbar von dem Pumpengehäuse gebildet werden. Vorzugsweise ist das Reservoir jedoch in Form eines Behältnisses, insbesondere einer Ampulle, gebildet, die von dem Pumpengehäuse aufgenommen wird, vorzugsweise austauschbar. Ferner lagert das Pumpengehäuse die Fördereinrichtung, die vorzugsweise einen Motor für die Erzeugung einer Förderbewegung umfasst. Das Pumpengehäuse bildet zusammen mit den von ihm aufgenommenen Komponenten der Infusionspumpe ein erstes Modul, nämlich einen Pumpenkopf. Vorzugsweise sind die von dem Pumpengehäuse aufgenommenen Komponenten in dem Pumpengehäuse untergebracht. Als aufgenommen werden im Sinne der Erfindung jedoch grundsätzlich auch solche Komponenten verstanden, die an dem Pumpengehäuse außen befestigt oder zumindest nicht gänzlich von dem Pumpengehäuse eingeschlossen sind. Allerdings sind das gänzliche Einschließen und in diesem Sinne verstandene Unterbringung in dem Pumpengehäuse die bevorzugte Art der Anordnung und Lagerung der Komponenten des Pumpenkopfs, soweit solche Komponenten nicht unumgänglich aus dem Pumpengehäuse herausgeführt sein müssen.

Für die modulare Aufspaltung umfasst die Infusionspumpe ein weiteres Gehäuse, das insbesondere eine Steuerungseinrichtung für die Fördereinrichtung, vorzugsweise für einen Motor der Fördereinrichtung, und/oder eine Bedienungseinrichtung und/oder eine Anzeigeeinrichtung der Infusionspumpe aufnehmen kann, wie dies grundsätzlich aus dem Stand der Technik bekannt ist.

Nach der Erfindung nimmt das weitere Gehäuse jedoch in jedem Fall die Energiequelle für die Fördereinrichtung auf. Die für den Betrieb der Fördereinrichtung erforderliche Energie wird vorzugsweise leitungsgebunden zwischen dem weiteren Gehäuse und dem Pumpengehäuse übertragen. Statt dessen wäre jedoch auch eine drahtlose Energieübertragung möglich, insbesondere über kürzere Entfernungen von etwa 50 cm oder weniger.

Im Vergleich zu herkömmlichen Infusionspumpen, die sämtliche Pumpenkomponenten in einem einzigen Gehäuse vereinen, bietet die modulare Infusionspumpe nach der Erfindung bereits den Vorteil, dass das Reservoir und die Fördereinrichtung ständig in unmittelbarer Nähe des Ortes der Verabreichung, im Allgemeinen einer Einstichstelle, angebracht werden und dort zumindest bis zu einem Austausch oder Nachfüllen des Reservoirs verbleiben können. Im Vergleich mit den bekannten modularen Infusionspumpen, die diesen Vorteil grundsätzlich ebenfalls bieten, können das Gewicht und die Größe des Pumpenkopfs nochmals reduziert werden. Es kommen hierbei nicht nur der Wegfall des Gewichts und des Volumens allein der Energiequelle vorteilhaft zum tragen. Durch das Herauslösen der Energiequelle aus dem Pumpenkopf kann das Pumpengehäuse vereinfacht und auch am Pumpengehäuse selbst Gewicht und Volumen eingespart werden.

In bevorzugten Ausführungen sind Teile einer Steuerungseinrichtung der Infusionspumpe oder es ist vorzugsweise die gesamte Steuerungseinrichtung aus dem Pumpenkopf ausgelagert. Die Steuerungseinrichtung oder die ausgelagerten Teile kann oder können insbesondere von dem weiteren Gehäuse aufgenommen werden. Die Steuerungseinrichtung untergliedert sich in einen Leistungsteil, über den die Energiequelle die Fördereinrichtung mit der Energie versorgt, und einen Signalverarbeitungsteil, der den Leistungsteil und über den Leistungsteil die Fördereinrichtung steuert. Die Steuerungseinrichtung ist vorzugsweise zu einer Steuerungs- und Regelungseinrichtung weitergebildet, wird im Folgenden der Einfachheit wegen jedoch stets nur als Steuerungseinrichtung bezeichnet. Von der Steuerungseinrichtung ist vorzugsweise wenigstens entweder der Leistungsteil oder der Signalverarbeitungsteil oder bevorzugter der Leistungsteil und der Signalverarbeitungsteil aus dem Pumpengehäuse, d.h. aus dem Pumpenkopf, ausgelagert.

Von dem Energieversorgungssystem der Fördereinrichtung ist oder sind im Pumpengehäuse vorzugsweise nur die Leitung oder die mehreren Leitungen für die Energieübertragung angeordnet. Vorzugsweise wird über diese Leitung oder diese mehrere Leitungen die Fördereinrichtung auch gesteuert und vorteilhafterweise geregelt.

Falls die Steuerungseinrichtung die Fördereinrichtung wie bevorzugt auch regelt und dementsprechend einen Positions- oder/und Geschwindigkeitsgeber umfasst, der eine Position oder/und Geschwindigkeit einer Komponente der Fördereinrichtung, vorzugsweise des genannten Motors, detektiert, so nimmt das Pumpengehäuse über den Geber hinaus vorzugsweise lediglich die für die Energieversorgung und/oder Signalübertragung des Gebers erforderlichen Leitungen auf, gegebenenfalls nur eine einzige Leitung für die kombinierte Übertragung von Energie und Signalen.

Sollte, wie in bevorzugten Ausführungen, in dem Pumpengehäuse ein Okklusions- und/oder Leckdetektor angeordnet sein, so gilt bezüglich der Energieversorgung und Signalübertragung dieses Detektors das vorstehend für den Positions- oder Geschwindigkeitsgeber Gesagte.

In besonders bevorzugter Ausführung dient das Pumpengehäuse somit als Träger nur für das Reservoir, stromabwärts von dem Reservoir befindliche Komponenten eines das Reservoir umfassenden produktführenden Systems, einen die Förderbewegung der Fördereinrichtung erzeugenden Antrieb, und die von dem Antrieb angetriebenen weiteren Komponenten der Fördereinrichtung. Gegebenenfalls ist das Pumpengehäuse auch noch der Träger eines oder mehrerer Geber für die Steuerung des Antriebs, falls dieser geregelt wird, und zusätzlicher Überwachungseinrichtungen, wie beispielsweise einer Okklusions- und/oder Lecküberwachungseinrichtung.

Der Antrieb kann insbesondere ein Motor sein, vorzugsweise ein elektrischer Drehmotor. Ein elektrischer Linearmotor ist grundsätzlich jedoch ebenfalls einsetzbar. Ganz grundsätzlich ist sogar denkbar, dass der Motor ein Pneumatik- oder Hydraulikmotor ist. Der Antrieb der Fördereinrichtung kann alternativ auch auf anderen Effekten beruhen, beispielsweise dem Piezoeffekt. Auch Mikrosystempumpen sind denkbar. Konventionelle Antriebe werden aber bevorzugt.

Die Energiequelle ist vorzugsweise eine elektrische Batterie oder ein elektrischer Akkumulator. Aber auch hier sind grundsätzlich Alternativen denkbar, beispielsweise die Ausbildung der Energiequelle als Druckreservoir, als Brennstoffzelle oder als zusätzliche Versorgungspumpe für die Versorgung eines Pneumatik- oder Hydraulikmotors mit dem Druckfluid.

Nach einem weiteren Aspekt der Erfindung ragt eine Infusionskanüle nur mit einer in oder unter die Haut einzubringenden Kanülenlänge über die Unterseite des Pumpengehäuses hinaus. Dieses Merkmal wird beispielsweise auch dann als erfüllt angesehen, wenn die Infusionskanüle seitlich aus dem Pumpengehäuse herausgeführt und an einer Seitenwand des Pumpengehäuses entlang bis auf das Niveau der Unterseite geführt und anschließend über die Unterseite hinaus um die besagte Kanülenlänge verlängert ist. Besonderes bevorzugt ragt die Infusionskanüle jedoch nur mit der besagten Kanülenlänge aus dem Gehäuse heraus. Eine Verbindungsleitung, die das Reservoir mit der Infusionskanüle verbindet, ist vorzugsweise 5 cm lang und ist noch bevorzugter kürzer als 5 cm.

Indem die in Körpergewebe einzubringende Infusionskanüle unmittelbar bei oder vorzugsweise sogar an dem Pumpengehäuse vorgesehen ist, wird sichergestellt, dass das Reservoir und ein in dem Körpergewebe befindlicher Auslass der Infusionskanüle die gleiche hydrostatische Höhe oder allenfalls eine für die praktischen Belange vernachlässigbare Höhendifferenz aufweisen. Ein Syphoning, d. h. eine Saugsituation in dem Reservoir mit der Folge der unkontrollierten Produktausschüttung, kann somit nicht entstehen. Ein besonderer Vorteil ist ferner, dass Produktverluste, die mit einem Priming der Infusionspumpe verbunden sind, minimiert werden. Als Priming bezeichnet man den Vorgang, durch den das von dem Reservoir bis zu dem Auslass der Infusionskanüle reichende Produktsystem entlüftet wird. Des Weiteren trägt die Verkürzung des Produktsystems zur Verringerung des Risikos von Okklusionen und/oder Leckagen im System bei. Durch den Verzicht auf einen aus dem Gehäuse herausgeführten Katheter, der bei herkömmlichen Infusionspumpen bis zu der Infusionskanüle reicht, kann schließlich auch die Zahl der von dem Verwender zu bevorratenden und auf Reisen mitzuführenden Einwegartikel verringert werden.

Das Reservoir kann einen Produktauslass an der Unterseite des Pumpengehäuses bilden, so dass die Infusionskanüle sich unmittelbar an den Reservoirauslass anschließen kann. Im Falle der bevorzugten Förderung des Produkts mittels eines Kolbens, der in dem Reservoir auf einen Reservoirauslass zu bewegbar aufgenommen ist, wird der Reservoirauslass in den meisten Fällen in die Bewegungsrichtung des Kolbens und daher meist winkelig; im Allgemeinen rechtwinklig, zu der Unterseite des Pumpengehäuses weisen. In solch einer bevorzugten Ausführungsform überbrückt die Verbindungsleitung auch den Winkel zwischen dem Reservoirauslass und der Infusionskanüle.

Das Pumpengehäuse ist erfindungsgemäß mehrteilig, besonders bevorzugt zweiteilig, und umfasst ein das Reservoir aufnehmendes oder selbst bildendes Hauptgehäuse und ein Nebengehäuse, aus dem die Infusionskanüle herausragt und das an dem Hauptgehäuse befestigt ist. Das Nebengehäuse ist vorteilhafterweise von Hand ohne Hilfsmittel lösbar an dem Hauptgehäuse befestigt. Besonders bevorzugt ist die Verbindung nach dem Lösen mit einem oder mehreren einfachen Handgriffen wiederherstellbar. Das Hauptgehäuse und das Nebengehäuse sind miteinander mittels einer Kupplung verbunden, beispielsweise in Form einer Schraub- oder bevorzugter einer Bajonettverbindung oder einer einfachen Steckverbindung oder einer Rastverbindung oder auch einer kombinierten Steck- und Rastverbindung. Das Haupt- und das Nebengehäuse können auch bei Ausbildung der Kupplung als Schraub- oder Bajonettverbindung zusätzlich miteinander verrasten.

Das Nebengehäuse bildet vorzugsweise einen Winkeladapter, der einen Winkel zwischen dem Reservoirauslass und der Infusionskanüle überbrückt

Das Nebengehäuse kann um wenigstens eine Drehachse drehbeweglich mit dem Hauptgehäuse verbunden sein, um die Infusionskanüle von Drehbewegungen des Hauptgehäuses um die Drehachse zu entkoppeln. Die Befestigung an dem Hauptgehäuse kann auch so gestaltet sein, dass das Hauptgehäuse relativ zu dem Nebengehäuse Drehbewegungen um zwei oder auch drei Drehachsen ausführen kann, so dass eine noch weitergehende Entkopplung erzielt wird. Denkbar ist auch, dass das Nebengehäuse an dem Hauptgehäuse translativ um ein kleines Wegstück bewegbar geführt ist. In bevorzugter, besonders einfacher Ausführung ist das Nebengehäuse an dem Hauptgehäuse jedoch steif befestigt, bevorzugt mittels der lösbaren Kupplung.

Weitere bevorzugte Merkmale der Erfindung werden in den Unteransprüchen und durch die Kombinationen der Ansprüche beschrieben.

Ein Ausführungsbeispiel der Erfindung wird nachfolgend anhand von Figuren erläutert. An dem Ausführungsbeispiel offenbar werdende Merkmale bilden je einzeln und in jeder Merkmalskombination die Gegenstände der Ansprüche und die vorstehend beschriebenen Ausgestaltungen vorteilhaft weiter. Es zeigen:
- Figur 1: eine modulare Infusionspumpe nach der Erfindung,
- Figur 2: einen Pumpenkopf der Infusionspumpe in einer Seitenansicht,
- Figur 3: den Pumpenkopf in einer Frontansicht und
- Figur 4: den Pumpenkopf in einer Draufsicht.

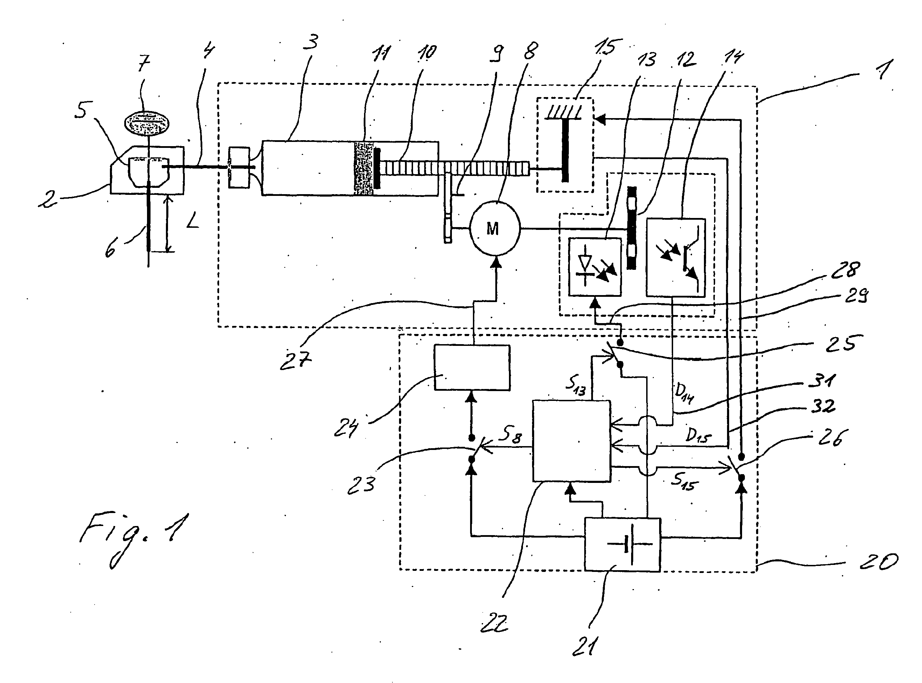

Figur 1 zeigt eine Infusionspumpe, die aus zwei Modulen besteht. Ein erster der beiden Module wird unmittelbar an einem Infusionsort unmittelbar auf der Haut getragen und im Folgenden als Pumpenkopf bezeichnet. Der Pumpenkopf ist in einem zweiteiligen Pumpengehäuse untergebracht, das mit einer Unterseite auf der Haut aufliegt und für solch eine Anbringung mit entsprechenden Befestigungsmitteln, beispielsweise einem Klebepad oder einem Gurt, ausgestattet ist. Das Pumpengehäuse besteht aus einem Hauptgehäuse 1 und einem Nebengehäuse 2, die mechanisch mittels eines Kupplungsmechanismus miteinander verbunden sind, so dass sie von Hand und ohne Hilfsmittel einfach und rasch voneinander lösbar und anschließend wieder miteinander verbindbar sind.

In dem Hauptgehäuse 1 sind ein Reservoir 3, das mit einem fluiden Produkt gefüllt ist, eine Fördereinrichtung 8-11 für die Förderung des Produkts, ein der Fördereinrichtung 8-11 zugeordneter Positionsgeber 12-14 und ferner ein Okklusions- und Leckdetektor 15 untergebracht. Das fluide Produkt kann insbesondere eine Medikamentenflüssigkeit sein, wie beispielsweise Insulin. Die Fördereinrichtung 8-11 ist konventionell als Kolbenpumpe mit Spindeltrieb gebildet. Die für die Produktförderung erforderliche Kraft wird mittels eines Elektromotors 8 erzeugt, dessen Antriebsgeschwindigkeit mittels eines Zahnradgetriebes 9 untersetzt auf eine in dem Hauptgehäuse 1 axial geradgeführte Kolbenstange 10 übertragen wird. Die Kolbenstange 10 ist als Gewindestange gebildet. Ein Ausgangszahnrad des Getriebes 9 ist mit dem Gewinde der Kolbenstange 10 in einem Gewindeeingriff. Aufgrund ihrer Geradführung wird die Kolbenstange 10 somit bei einem Drehantrieb des Ausgangszahnrads des Getriebes 9 axial in eine Vortriebsrichtung bewegt. Die Kolbenstange 10 drückt hierbei gegen einen in dem Reservoir 3 aufgenommenen Kolben 11, der hierdurch in dem Reservoir 3 in Richtung auf einen Auslass des Reservoirs 3 zu bewegt wird, wobei die Richtung der Bewegbarkeit des Kolbens 11 mit der Vortriebsrichtung der Kolbenstange 10 identisch ist. Ein Vortrieb der Kolbenstange 10 bewirkt einen ebensolchen Vortrieb des Kolbens 11 und damit die Verdrängung von Produkt durch den Auslass des Reservoirs 3. Von der Fördereinrichtung 8-11 sind somit der Motor 8 und alle in dem von dem Motor 8 ausgehenden Kraftfluss abwärts angeordneten Komponenten bis zu dem unmittelbar auf das Produkt wirkenden Förderglied, nämlich der Kolben 11, untergebracht.

Das Produkt wird durch eine flexible Infusionskanüle 6 verabreicht. Die Infusionskanüle 6 ragt von der Unterseite des Nebengehäuses 2 ab, im Ausführungsbeispiel ragt sie durch eine die Unterseite des Nebengehäuses 2 bildende Gehäusewand hindurch. Die Infusionskanüle 6 wird für eine subkutane Verabreichung mit ihrer gesamten, über die Unterseite des Nebengehäuse 2 vorragenden Kanülenlänge 1 in das Körpergewebe eingebracht. Die Kanülenlänge 1 ist die Länge der Infusionskanüle 6 gemessen zwischen der Unterseite des Nebengehäuses 2 und dem vorderen freien Ende der Infusionskanüle 6.

Abgesehen von den produktführenden Teilen von dem Reservoir 3 bis zu der Infusionskanüle 6, dem Motor 8 und den im Kraftfluss abwärts von dem Motor 8 angeordneten Komponenten der Fördereinrichtung 8-11 und den beiden Sensoren 12-14 und 15 umfasst der Pumpenkopf nur noch Leitungen für die Energieversorgung dieser Komponenten und für die Signalübertragung zu und von diesen Komponenten. Alle weiteren Komponenten der Infusionspumpe sind in ein weiteres Modul mit einem separaten, weiteren Gehäuse 20 ausgelagert. Das weitere Modul ist ein Servicemodul, in das insbesondere die Energieversorgung für sämtliche Komponenten des Pumpenkopfs ausgelagert ist. Ausgelagert sind ferner auch sämtliche Funktionen der Steuerung und Regelung der Komponenten des Pumpenkopfs. Soweit aufgrund der Rückführung einer oder mehrerer Regelgrößen nicht nur gesteuert, sondern auch geregelt wird, wird im Folgenden vereinfachend nur über das Steuern und dementsprechend über eine Steuerungseinrichtung oder kurz eine Steuerung gesprochen. Über die beiden Funktionen der Energieversorgung und Steuerung hinaus sind auch sämtliche Bedienungsfunktionen und auch alle optischen und/oder akustischen Anzeigefunktionen in das Servicemodul ausgelagert. Der Pumpenkopf enthält somit nur noch die für die Förderung des Produkts und die Überwachung der ordnungsgemäßen Förderung erforderlichen Komponenten der Infusionspumpe und hat deshalb ein besonders geringes Gewicht und ein besonders geringes Volumen. Aufgrund dieser beiden Eigenschaften ist der Pumpenkopf für die Anbringung unmittelbar am Infusionsort in besonderer Weise geeignet. Wegen der Anbringung unmittelbar am Körper kann in dem Pumpengehäuse 1/2 vorteilhafterweise allerdings ein Vibrationsalarm aufgenommen sein.

In dem Gehäuse 20 des Servicemoduls sind insbesondere untergebracht eine Energiequelle 21, die sämtliche Komponenten der Infusionspumpe mit der benötigten Energie versorgt. Im Ausführungsbeispiel ist die Energiequelle 21 eine Batterie oder ein elektrischer Akku. Dargestellt ist ferner eine Steuerungseinrichtung mit ihren wesentlichen Eingängen und Ausgängen. Die Steuerungseinrichtung umfasst einen Signalverarbeitungsteil 22 und speziell für den Motor 8 einen Leistungsteil 24, im Ausführungsbeispiel eine Leistungselektronik. Ferner umfasst sie mehrere Unterbrecher, nämlich einen Unterbrecher 23 in der Energieversorgungsleitung für den Motor 8, einen Unterbrecher 25 in der Energieversorgungsleitung für den Positionsgeber 12-14 und einen Unterbrecher 26 in der Energieversorgungsleitung für den Okklusions- und Leckdetektor 15. Alle drei Energieversorgungsleitungen gehen von der gemeinsamen Energiequelle 21 aus. Selbstverständlich kann die Energiequelle 21 auch erst von mehreren separaten Energiequellen in Kombination gebildet werden, vorzugsweise ist die Energiequelle 21 jedoch einheitlich eine einzige Batterie oder ein einziger Akku. Der Signalverarbeitungsteil 22 steuert die Unterbrecher 23, 25 und 26 je mittels eines eigenen Stellsignals, nämlich mittels eines Stellsignals S₈ für den Unterbrecher 23, eines Stellsignals S₁₃ für den Unterbrecher 25 und eines Stellsignals S₁₅ für den Unterbrecher 26. An den Eingang des Signalverarbeitungsteils 22 werden die Ausgangssignale der beiden Geber bzw. Detektoren 12-14 und 15 gelegt. Das Ausgangssignal des Positionsgebers 12-14 ist mit D₁₄ und das Ausgangssignal des Okklusions- und Leckdetektors 15 ist mit D₁₅ bezeichnet.

Das Ausgangssignal D₁₄ repräsentiert die Drehwinkelposition des Motors 8. Zu der Detektion der Drehwinkelposition umfasst der Positionsgeber 12-14 eine Lochscheibe, die drehfest auf einer Welle des Motors 8 angebracht ist. Zur einen axialen Seite der Lochscheibe 12 ist eine Leuchtdiode 13 und zur anderen Seite ist ein Fototransistor 14 gehäusefest angeordnet. Der Fototransistor 14 empfängt das die Lochscheibe 12 durchdringende Licht der Leuchtdiode 13 und gibt es als Ausgangssignal D₁₄ über eine Signalleistung 31 auf den Eingang des Signalverarbeitungsteils 22. Das Ausgangssignal D₁₄ des Positionsgebers 12-14 bildet die Regelgröße für die Steuerung des Motors 8. Der Signalverarbeitungsteil 22 generiert entsprechend einer vorgegebenen Programmierung die Führungsgröße, vergleicht sie mit der Regelgröße D₁₄ und bildet in Abhängigkeit von dem Vergleich das Stellsignal S₈ für den Unterbrecher 23. Der Unterbrecher 23 nimmt je nach Stellsignal 8 eine von zwei möglichen Schaltstellungen ein. In der einen Schaltstellung schließt er die Leitung für die Energieversorgung des Motors 8 und im anderen Fall unterbricht er sie. Die Motorsteuerung kann konventionell gebildet sein und bedarf daher keiner weiteren Erläuterung, als lediglich des Hinweises, dass alle ihre Komponenten mit Ausnahme der Leitung 27 für die Energieversorgung des Motors 8 Komponenten des Servicemoduls und nicht des Pumpenkopfs sind. Aufgrund der schlupffreien Kraftübertragung vom Motor 8 auf die Kolbenstange 10 repräsentiert die Drehwinkelposition des Motors 8 gleichzeitig auch die Position der Kolbenstange 10 und damit des Kolbens 11 in dem Reservoir 3. Aus der Drehwinkelposition des Motors 8 kann daher unmittelbar auf die geförderte Produktmenge und die im Reservoir 3 verbliebene Restmenge geschlossen werden. Mittels des Positionsgebers 12-14 kann grundsätzlich auch die Drehzahl und damit die Drehgeschwindigkeit des Motors 8 und in der Folge die Vortriebsgeschwindigkeit des Kolbens 11 und somit die Fördergeschwindigkeit bzw. Förderrate ermittelt werden. Unumgänglich erforderlich ist dies jedoch nicht.

Der zum Zwecke der Ernergieeinsparung in der Energieversorgungsleitung für die Leuchtdiode 13 angeordnete Unterbrecher 25 ist ebenfalls ein einfacher Schalter mit zwei Stellungen, der in der einen der beiden Stellungen die Energieversorgungsleitung schließt und in der anderen unterbricht. Auch der Unterbrecher 25 ist eine Komponente des Servicemoduls und ist in dessen Gehäuse 20 angeordnet. In dem Hauptgehäuse 1 des Pumpenkopfs sind lediglich das Reststück 28 der Energieversorgungsleitung für die Leuchtdiode 13 und das Reststück der Signalleitung 31 untergebracht.

Mittels des Okklusions- und Leckdetektors wird die auf die Kolbenstange 10 bei der Förderung wirkende Reaktionskraft gemessen, und das Ausgangssignal D₁₅ repräsentiert die gemessene Reaktionskraft. Der Okklusions- und Leckdetektor 15 dient ausschließlich dem Zweck der Überwachung. Stellt der Signalverarbeitungsteil 22 anhand des Ausgangssignals D₁₅ das Auftreten einer Okklusion oder eines Lecks in dem das Produkt führenden Teil der Infusionspumpe fest, so unterbricht der Signalverarbeitungsteil 22 mittels eines entsprechenden Stellsignals S₈ die Energieversorgung für den Motor 8 und setzt die Fördereinrichtung 8-11 dadurch fest.

Als weitere Überwachungseinrichtung kann selbstverständlich auch der Positionsgeber 12-14 dienen. Auch in Abhängigkeit von dessen Ausgangssignal D₁₄ könnte beispielsweise mittels eines Vibrationsalarms ein vibratorisches Alarmsignal ausgegeben werden.

Im Ausführungsbeispiel wird der Okklusions- und Leckdetektor 15 ebenfalls mit Energie versorgt, nämlich über eine eigene Energieversorgungsleitung 29 von der Energiequelle 21. In der Energieversorgungsleitung ist der Unterbrecher 26 angeordnet, der ebenfalls als einfacher Schalter mit zwei möglichen Stellungen gebildet ist. Was die Energieversorgung des Detektors 15 und dessen Ausgangssignal anbetrifft, so sind in dem Pumpengehäuse 1/2 wieder nur die Reststücke der betreffenden Leitungen 29 und 32 angeordnet, um Volumen und Gewicht des Pumpenkopfs zu minimieren.

Die beiden Unterbrecher 25 und 26 dienen der Energieeinsparung und während des Motorbetriebs ihre jeweilige Schließstellung ein. Vorzugsweise werden sie unmittelbar mit dem Einschalten der Steuerung 22-26 in die Schließstellung und unmittelbar nach jedem Abschalten aus der Schließstellung in die unterbrechende Stellung gebracht. Falls der Okklusions und Leckdetektor 15 als Kraftsensor gebildet ist, kann er beispielsweise ein Dehnmesssensor sein oder stattdessen beispielsweise als piezoelektrischer Aufnehmer gebildet sein und dementsprechend ohne Energieversorgung auskommen. Falls der Okklusions- und Leckdetektor ohne Fremdenergie auskommt, können vorteilhafterweise die für ihn vorgesehene Energieversorgungsleitung 29, der Unterbrecher 26 und die Signalleitung oder der Signalsender für das Stellsignal S₁₅ eingespart werden.

Falls der Pumpenkopf über einen Vibrationsalarm verfügt, wie dies bevorzugt wird, so würde solch ein Vibrationsalarm ebenfalls wie beispielsweise der Positionsgeber 12 bis 14 von der Energiequelle 21 mit elektrischer Energie versorgt werden. In der Versorgungsleitung wäre ein weiterer Unterbrecher in der Art der Unterbrecher 23 bis 26 angeordnet und würde von dem Signalverarbeitungsteil 22 durch ein entsprechendes Stellsignal so gesteuert werden, dass die Energieversorgungsleitung zu dem Vibrationsalarm geschlossen und das vibratorische Alarmsignal ausgelöst würde. Aufgrund der Anbringung des Pumpenkopfs unmittelbar auf der Haut ist ein vibratorischer Alarm besonders wirksam.

Ein weiteres besonders vorteilhaftes Merkmal des Pumpenkopfs ist die kurze Länge der Produktdurchleitung bis zu der Infusionskanüle 6, wobei diese Länge zwischen dem Auslass des Reservoirs 3 und dem stromaufwärtigen Ende einer Kanülenlänge L gemessen wird. Die Kanülenlänge L ist die Länge, mit der die Infusionskanüle 6 während der Verabreichung des Medikaments in das Körpergewebe ragt. Bei in das Körpergewebe eingebrachter Kanülenlänge L handelt es sich bei der Länge der Produktdurchleitung um die zwischen dem Auslass des Reservoirs 3 und der Hautoberfläche gemessene Länge. Diese Länge beträgt nicht mehr als 5 cm, vorzugsweise nicht mehr als 2 cm, und kann sogar noch kleiner sein.

Die Produktdurchleitung bilden eine Verbindungskanüle 4, ein von einem Diaphragma 5 umschlossener Hohlraum und ein stromaufwärtiges Endstück der Infusionskanüle 6, mit dem die Infusionskanüle 6 in das Nebengehäuse 2 ragt. Die Verbindungskanüle 4 durchragt ein Septum, das den Auslass des Reservoirs 3 verschließt. Mit ihrem gegenüberliegenden stromabwärtigen Ende durchragt die Verbindungskanüle 4 eine Seitenwand des Diaphragmas 5 und ragt dort in den Hohlraum des Diaphragmas 5 hinein. Stromabwärts von der Verbindungskanüle 4 ragt die Infusionskanüle 6 mit ihrem stromaufwärtigen Ende ebenfalls in den Hohlraum hinein, so dass von dem Auslass des Reservoirs 3 bis in die Infusionskanüle 6 eine fluiddichte Produktdurchleitung geschaffen wird.

Da die Infusionskanüle 6 im Ausführungsbeispiel flexibel und insbesondere nicht biegesteif gebildet ist, wird sie mit Hilfe einer Einstechnadel 7 in das Körpergewebe, d. h. in die Haut oder vorzugsweise durch die Haut, eingebracht. Die Einstechnadel 7 weist eine für das Einstechen ausreichende Steifigkeit auf. Die Infusionskanüle 6 umgibt die Einstechnadel 7 eng anliegend, so dass sie bei dem Einstechen der Einstechnadel 7 mit in das Körpergewebe eingebracht wird. Die Einstechnadel 7 wird nach dem Einbringen der Infusionskanüle 6 aus dem Körpergewebe und der Infusionskanüle 6 sowie dem Diaphragma 5 herausgezogen und entfernt. Das Diaphragma 5 dichtet nach dem Herausziehen der Einstechnadel 7 weiterhin den die Verbindungskanüle 4 und die Infusionskanüle 6 verbindenden Hohlraum fluiddicht ab.

Für das Einbringen der Infusionskanüle 6 in das Körpergewebe ist es vorteilhaft, wenn das Nebengehäuse 2 wie im Ausführungsbeispiel mit dem Hauptgehäuse 1 des Pumpenkopfs so verbunden ist, dass mit der Anbringung auf der Haut gleichzeitig auch die Infusionskanüle 6 in die Haut oder durch die Haut in das Körpergewebe eingebracht wird und hierfür kein zusätzlicher Handgriff außer der Anbringung des Pumpengehäuses 1/2 erforderlich ist. In bevorzugter einfacher Ausführung ist das Nebengehäuse 2 wie im Ausführungsbeispiel vollkommen starr, d. h. unbeweglich, mit dem Hauptgehäuse 1 verbunden, beispielsweise mittels eines Bajonettverschlusses oder eines anderen geeigneten Verschlusses als verbindende Kupplung. Das Hauptgehäuse 1 und das Nebengehäuse 2 bilden vorzugsweise eine gemeinsame glatte Unterseite für die Anbringung auf der Haut. Das Nebengehäuse 2 bildet ferner einen Adapter, der den Winkel zwischen dem Auslass des Reservoirs 3 und der Hautoberfläche überbrückt.

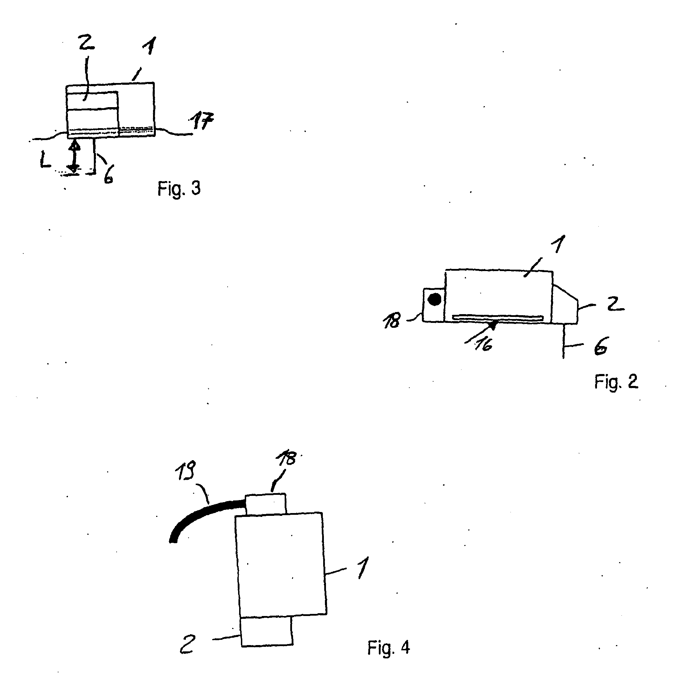

Die Figuren 2, 3 und 4 zeigen den Pumpenkopf in unterschiedlichen Ansichten auf das Pumpengehäuse 1/2. Die Infusionskanüle 6 ist mit ihrer Kanülenlänge L in das Körpergewebe eingebracht worden. Für die Anbringung des Pumpengehäuses 1/2 auf der Haut ist in Figur 2 ein nahe bei der Unterseite des Pumpengehäuses 1/2 gebildeter Schlitz für ein Befestigungsmittel 17, beispielsweise ein Klebepad oder ein Befestigungsgurt, zu erkennen. Figur 3 zeigt in einer Frontansicht auf die Seite des Nebengehäuses 2 ein durch den Schlitz 16 gezogenes Klebepad, das beidseits durch den Schlitz 16 über das Hauptgehäuse 1 hinausragt und mit seinen beiden freien Enden auf der Hautoberfläche aufgeklebt werden kann. Die Infusionskanüle 6 weist senkrecht zu der im Ausführungsbeispiel planen, am Infusionsort auf der Haut sitzenden Unterseite des Pumpengehäuses 1/2.

In den Figuren 2 und 4 ist ferner auch ein Steckverbinder 18 zu erkennen, der in einen Steckanschluss des Hauptgehäuses 1 eingesteckt ist. Über den Steckverbinder 18 und das davon ausgehende Verbindungskabel 19 werden die gesamte Energie und sämtliche Signale zu und von dem Pumpenkopf übertragen. Im Ausführungsbeispiel findet die Übertragung ausschließlich zwischen dem Pumpenkopf und dem Servicemodul statt. Der Steckverbinder 18 ist als Winkelsteckverbinder gebildet. Das Verbindungskabel 19 verlässt den Steckverbinder 18 in einem rechten Winkel zu dem Anschluss des Pumpenkopfs. Die Ausbildung als Winkelsteckverbinder wird gegenüber einem grundsätzlich ebenfalls möglichen, geraden axialen Anschluss bevorzugt, da hierdurch die bei Körperbewegungen auf die Infusionskanüle 6 wirkenden Querkräfte verringert werden können. Der Steckverbinder 18 ist wasserdicht. Das Verbindungskabel 19 ist eine hochflexible und geschirmte elektrische Leitung. Die Verbindungsleitung 19 ist entweder integraler Bestandteil der Steuerung 22-26 oder ist mit der Steuerung 22-26 ebenfalls über einen Steckverbinder und einen entsprechenden Anschluss am Gehäuse 20 gekoppelt.

Die Integrität und korrekte Funktion der Infusionspumpe kann aufgrund der Energieversorgungs- und Signalverbindung leichter als bei konventionellen Infusionspumpen, bei denen von der Infusionspumpe ein langer Verbindungskatheter bis zum Infusionsort reicht, überwacht werden, im einfachsten Fall beispielsweise durch eine Messung der Impedanz, wobei die Impedanzmessung permanent oder vorzugsweise in regelmäßigen Intervallen durchgeführt wird. Mittels der Überwachung der Energieversorgungs- und Signalverbindung ist eine Detektion und auch Unterscheidung relevanter Fehlersituationen, z. B. eine Unterbrechung oder ein Kurzschluss, etwa durch eindringende Feuchtigkeit, möglich. Gegenüber bekannten Pumpenköpfen, die ebenfalls unmittelbar auf der Haut angebracht werden, fällt als Vorteil zumindest noch die verkürzte Produktdurchleitung zwischen dem Auslass des Reservoirs 3 und der in das Körpergewebe eingebrachten Kanülenlänge 1 der Infusionskanüle 6 ins Gewicht.

Als noch ein weiterer, immanenter Vorteil des Pumpenkopfs ist die Möglichkeit zu nennen, dass ein Durchflusssensor zum einen in unmittelbarer Nähe der in das Körpergewebe eingebrachten Kanülenlänge L angeordnet werden kann und zum anderen die signaltechnische und, falls erforderlich, die für die Energieversorgung des Durchflusssensors erforderliche Verbindung nicht über flexible Durchleitungslcatheter, sondern über das zwar mehrteilige, aber im verbundenen Zustand einheitliche Pumpengehäuse 1/2 vorgenommen werden kann. Dies ist nicht nur vorteilhaft im Falle einer leitungsgebundenen Verbindung, sondern auch im Falle einer drahtlosen Anbindung des Durchflusssensors. Die Anbindung und Einbindung eines Durchflusssensors in die Steuerung 22-26 würde sich ebenso gestalten, wie beispielsweise die Anbindung und Einbindung des Okklusions- und Leckdetektors 15.

Das Nebengehäuse 2 ist mit sämtlichen von ihm aufgenommenen Komponenten des Pumpenkopfs als Einwegartikel konzipiert. Dieser Einwegartikel kann beispielsweise jedes Mal dann getauscht werden, wenn das Reservoir nachgefüllt oder vorzugsweise im Ganzen ausgetauscht wird. Der Lebenszyklus des Einwegartikels kann jedoch auch länger sein. Zu dem Einwegartikel gehören die in dem Nebengehäuse 2 untergebrachten Komponenten und natürlich ebenso die Infusionskanüle 6 und vorzugsweise auch die Verbindungskanüle 4. Bei der Befestigung des Nebengehäuses 2 an dem Hauptgehäuse 1 wird gleichzeitig auch die Verbindung mit dem Reservoir 3 hergestellt.

## Patentansprüche

1. Modulare Infusionspumpe für die Verabreichung eines Produkts, die Infusionspumpe umfassend:
a) ein Pumpengehäuse (1, 2), das mit einer Unterseite unmittelbar auf menschlicher oder tierischer Haut angebracht werden kann,
b) ein von dem Pumpengehäuse (1, 2) aufgenommenes oder direkt gebildetes Reservoir (3) für das Produkt,
c) eine von dem Pumpengehäuse (1, 2) gelagerte Fördereinrichtung (8-11), mittels der das Produkt aus dem Reservoir (3) förderbar ist,
d) eine Energiequelle (21) für die Fördereinrichtung (8-11)
e) ein von dem Pumpengehäuse (1, 2) separates, während der Verarbreichung entfernt tragbares weiteres Gehäuse (20), das die Energiequelle (21) aufnimmt,
f) eine Übertragungseinrichtung (19) für die Übertragung von Energie der Energiequelle (21) zu der Fördereinrichtung (8-11),
g) und eine Infusionskanüle (6), welche nur mit einer in oder unter die Haut einzubringenden Kanülenlänge (L) über die Unterseite des Pumpengehäuses (1, 2) hinausragt,
h) wobei das Pumpengehäuse (1, 2) ein das Reservoir (3) aufnehmendes oder selbst bildendes Hauptgehäuse (1) und ein die Infusionskanüle (6) aufnehmendes Nebengehäuse (2) umfasst,
**dadurch gekennzeichnet, dass**
i) das Nebengehäuse (2) an dem Hauptgehäuse (1) mittels einer Kupplung lösbar befestigt ist.

2. Modulare Infusionspumpe nach dem vorhergehenden Anspruch, **dadurch gekennzeichnet, dass** die Infusionspumpe eine Steuerungseinrichtung (22-26) mit einem Leistungsteil (24) umfasst, über den die Energiequelle (21) die Fördereinrichtung (8-11) mit der Energie versorgt, und dass der Leistungsteil (24) von dem weiteren Gehäuse (20) aufgenommen wird.

3. Modulare Infusionspumpe nach einem der vorhergehenden Ansprüche, **dadurch gekennzeichnet, dass** die Infusionspumpe eine Steuerungseinrichtung (22-26) mit einem Unterbrecher (23) umfasst, um die Energieversorgung für die Fördereinrichtung (8-11) gesteuert zu unterbrechen, und dass der Unterbrecher (23) von dem weiteren Gehäuse (20) aufgenommen wird.

4. Modulare Infusionspumpe nach einem der zwei vorhergehenden Ansprüche, **dadurch gekennzeichnet, dass** in oder an dem Pumpengehäuse (1, 2) ein Detektor (12-14; 15) angeordnet ist, der eine physikalische Größe der Fördereinrichtung (8-11) oder eines produktführenden Teils detektiert und an die Steuerungseinrichtung (22-26) der Infusionspumpe ein die detektierte Größe repräsentierendes Ausgangssignal (D14; D15) ausgibt, in Abhängigkeit von dem die Steuerungseinrichtung (22-26) ein Stellsignal (S8) für die Fördereinrichtung (8-11) bildet.

5. Modulare Infusionspumpe nach dem vorhergehenden Anspruch, **dadurch gekennzeichnet, dass** die Energiequelle (21) den Detektor (12-14; 15) über einen Unterbrecher (25; 26), den die Steuerungseinrichtung (22-26) mittels eines Stellsignals (S13; S15) steuert, mit Energie versorgt.

6. Modulare Infusionspumpe nach einem der zwei vorhergehenden Ansprüche, **dadurch gekennzeichnet, dass** der Unterbrecher (25; 26) in dem weiteren Gehäuse (20) angeordnet ist.

7. Modulare Infusionspumpe nach einem der drei vorhergehenden Ansprüche, **dadurch gekennzeichnet, dass** der Detektor ein Positions- und/oder Geschwindigkeitsgeber (12-14) ist, der eine Position und/oder eine Geschwindigkeit der Fördereinrichtung (8-11) detektiert, und dass das Ausgangssignal (D14) des Detektors (12-14) die detektierte Position und/oder Geschwindigkeit repräsentiert.

8. Modulare Infusionspumpe nach einem der Ansprüche 4 bis 6, **dadurch gekennzeichnet, dass** der Detektor ein Okklusions- und/oder Leckdetektor (15) ist, der eine Okklusion und/oder ein Leck in einem sich von dem Reservoir (3) bis zu einem Auslass einer Infusionskanüle (6) erstreckenden, produktführenden Teil der Infusionspumpe detektiert.

9. Modulare Infusionspumpe nach einem der Ansprüche 4 bis 6, **dadurch gekennzeichnet, dass** der Detektor ein Durchflussdetektor ist, der unmittelbar vor einer Infusionskanüle (6) in einem produktführenden Teil der Infusionspumpe angeordnet ist.

10. Modulare Infusionspumpe nach einem der vorhergehenden Ansprüche, **dadurch gekennzeichnet, dass** von einem die Energiequelle (21) umfassenden Energieversorgungssystem für die Fördereinrichtung (8-11) nur eine oder mehrere Leitungen (27) für die Energieübertragung in oder an dem Pumpengehäuse (1, 2) angeordnet ist oder sind.

11. Modulare Infusionspumpe nach einem der vorhergehenden Ansprüche, **dadurch gekennzeichnet, dass** von einem die Energiequelle (21) umfassenden Energieversorgungssystem der Infusionspumpe nur Leitungen (27, 28, 29) für die Energieübertragung in oder an dem Pumpengehäuse (1, 2) angeordnet sind.

12. Modulare Infusionspumpe nach einem der Ansprüche 2 bis 11, **dadurch gekennzeichnet, dass** von einem die Energiequelle (21) und eine Steuerungseinrichtung (22-26) umfassenden Steuerungs- und Energieversorgungssystem für die Förderrichtung (8-11) nur Leitungen (27, 28, 29, 31, 32) für die Energieübertragung und/oder die Signalübertragung in oder an dem Pumpengehäuse (1, 2) angeordnet sind.

13. Modulare Infusionspumpe nach einem der vorhergehenden Ansprüche, **dadurch gekennzeichnet, dass** die Fördereinrichtung (8-11) einen Motor (8) und wenigstens ein von dem Motor angetriebenes Förderglied (11) umfasst, welches auf das Produkt wirkt und es dadurch fördert, und dass die Energiequelle (21) den Motor (8) mit der benötigten Energie versorgt.

14. Modulare Infusionspumpe nach einem der vorhergehenden Ansprüche, **dadurch gekennzeichnet, dass** das Pumpengehäuse (1, 2) und das weitere Gehäuse (20) mittels wenigstens einer Energieversorgungsleitung (27, 28, 29) miteinander verbunden sind.

15. Modulare Infusionspumpe nach dem vorhergehenden Anspruch, **dadurch gekennzeichnet, dass** die gesamte Energieübertragung zwischen dem Pumpengehäuse (1, 2) und dem weiteren Gehäuse (20) leitungsgebunden ist.

16. Modulare Infusionspumpe nach einem der vorhergehenden Ansprüche, **dadurch gekennzeichnet, dass** das Pumpengehäuse (1, 2) und das weitere Gehäuse (20) mittels wenigstens einer Signalleitung (31, 32) miteinander verbunden sind.

17. Modulare Infusionspumpe nach dem vorhergehenden Anspruch, **dadurch gekenzeichnet, dass** die gesamte Signalübertragung zwischen dem Pumpengehäuse (1, 2) und dem weiteren Gehäuse (20) leitungsgebunden ist.

18. Modulare Infusionspumpe nach einem der vorhergehenden Ansprüche, **dadurch gekennzeichnet, dass** die Infusionskanüle (6) von der Unterseite des Nebengehäuses (2) abragt.

19. Modulare Infusionspumpe nach einem der vorhergehenden Ansprüche, **dadurch gekennzeichnet, dass** das Nebengehäuse (2) an dem Hauptgehäuse (1) geführt bewegbar befestigt ist.

## Claims

1. A modular infusion pump for administering a product, said infusion pump comprising:
a) a pump casing (1, 2), a lower side of which can be placed directly on human or animal skin;
b) a reservoir (3) for the product, accommodated or directly formed by the pump casing (1, 2);
c) a conveying means (8-11) which is mounted by the pump casing (1, 2) and by means of which the product can be conveyed out of the reservoir (3);
d) an energy source (21) for the conveying means (8-11);
e) an additional casing (20) which is separate from the pump casing (1, 2) and can be worn at a distance during administering and which accommodates the energy source (21);
f) a transfer means (19) for transferring energy from the energy source (21) to the conveying means (8-11);
g) and an infusion cannula (6) which protrudes beyond the lower side of the pump casing (1, 2) only by a cannula length (L) to be introduced into or under the skin;
h) wherein the pump casing (1, 2) comprises a main casing (1) which accommodates the reservoir (3) or forms the reservoir (3) itself, and a secondary casing (2) which accommodates the infusion cannula (6),
**characterised in that**
i) the secondary casing (2) is detachably fastened to the main casing (1) by means of a coupling.

2. The modular infusion pump according to the preceding claim, **characterised in that** the infusion pump comprises a control means (22-26) comprising a power portion (24) via which the energy source (21) supplies energy to the conveying means (8-11), and **in that** the power portion (24) is accommodated by the additional casing (20).

3. The modular infusion pump according to any one of the preceding claims, **characterised in that** the infusion pump comprises a control means (22-26) comprising an interrupter (23) for interrupting the energy supply for the conveying means (8-11) in a controlled way, and **in that** the interrupter (23) is accommodated by the additional casing (20).

4. The modular infusion pump according to any one of the preceding two claims, **characterised in that** a detector (12-14; 15) is arranged in or on the pump casing (1, 2) and detects a physical parameter of the conveying means (8-11) or of a product-guiding portion and outputs an output signal (D14; D15) representing the detected parameter to the control means (22-26) of the infusion pump, in accordance with which the control means (22-26) forms an actuating signal (S8) for the conveying means (8-11).

5. The modular infusion pump according to the preceding claim, **characterised in that** the energy source (21) supplies energy to the detector (12-14; 15) via an interrupter (25; 26) which is controlled by the control means (22-26) by means of an actuating signal (S13; S15).

6. The modular infusion pump according to any one of the preceding two claims, **characterised in that** the interrupter (25; 26) is arranged in the additional casing (20).

7. The modular infusion pump according to any one of the preceding three claims, **characterised in that** the detector is a position and/or speed sensor (12-14) which detects a position and/or a speed of the conveying means (8-11), and **in that** the output signal (D14) of the detector (12-14) represents the detected position and/or speed.

8. The modular infusion pump according to any one of Claims 4 to 6, **characterised in that** the detector is an occlusion and/or leakage detector (15) which detects an occlusion and/or a leakage in a product-guiding portion of the infusion pump which extends from the reservoir (3) up to an outlet of an infusion cannula (6).

9. The modular infusion pump according to any one of Claims 4 to 6, **characterised in that** the detector is a flow detector which is arranged immediately in front of an infusion cannula (6) in a product-guiding portion of the infusion pump.

10. The modular infusion pump according to any one of the preceding claims, **characterised in that**, of an energy supply system comprising the energy source (21) for the conveying means (8-11), only one or more lines (27) for transferring energy is/are arranged in or on the pump casing (1, 2).

11. The modular infusion pump according to any one of the preceding claims, **characterised in that**, of an energy supply system of the infusion pump, comprising the energy source (21), only lines (27, 28, 29) for transferring energy are arranged in or on the pump casing (1, 2).

12. The modular infusion pump according to any one of Claims 2 to 11, **characterised in that**, of a control and energy supply system comprising the energy source (21) and a control means (22-26) for the conveying means (8-11), only lines (27, 28, 29, 31, 32) for transferring energy and/or transferring signals are arranged in or on the pump casing (1, 2).

13. The modular infusion pump according to any one of the preceding claims, **characterised in that** the conveying means (8-11) comprises a motor (8) and at least one conveying member (11) which is driven by the motor and acts on and thus conveys the product, and **in that** the energy source (21) supplies the motor (8) with the energy needed.

14. The modular infusion pump according to any one of the preceding claims, **characterised in that** the pump casing (1, 2) and the additional casing (20) are connected to each other by means of at least one energy supply line (27, 28, 29).

15. The modular infusion pump according to the preceding claim, **characterised in that** the entire energy transfer between the pump casing (1, 2) and the additional casing (20) is by lines.

16. The modular infusion pump according to any one of the preceding claims, **characterised in that** the pump casing (1, 2) and the additional casing (20) are connected to each other by means of at least one signal line (31, 32).

17. The modular infusion pump according to the preceding claim, **characterised in that** the entire signal transfer between the pump casing (1, 2) and the additional casing (20) is by lines.

18. The modular infusion pump according to any one of the preceding claims, **characterised in that** the infusion cannula (6) protrudes from the lower side of the secondary casing (2).

19. The modular infusion pump according to any one of the preceding claims, **characterised in that** the secondary casing (2) is fastened to the main casing (1) such that it can be moved and is guided.

## Revendications

1. Pompe de perfusion modulaire pour l'administration d'un produit, la pompe de perfusion comprenant:
a) un boîtier de pompe (1, 2) réalisé de sorte à pouvoir placer sa face inférieure sur la peau humaine ou animale,
b) un réservoir (3) pour le produit, formé directement ou reçu par le boîtier de pompe (1, 2),
c) un dispositif de circulation (8-11) logé par le boîtier de pompe (1, 2), à l'aide duquel le produit peut être extrait du réservoir (3),
d) une source d'énergie (21) pour le dispositif de circulation (8-11),
e) un autre boîtier (20) portable séparé du boîtier de pompe (1, 2) et éloigné pendant l'administration, qui reçoit la source d'énergie (21),
f) un dispositif de transmission (19) pour la transmission d'énergie de la source d'énergie (21) au dispositif de circulation (8-11),
g) et une canule de perfusion (6), qui ne dépasse de la face inférieure du boîtier de pompe (1, 2) que de la longueur de canule (L) à introduire dans ou sous la peau,
h) dans laquelle le boîtier de pompe (1, 2) comprend un boîtier principal (1) formant lui-même ou recevant le réservoir (3) et un boîtier auxiliaire (2) recevant la canule de perfusion (6),
**caractérisée en ce que**
i) le boîtier auxiliaire (2) est fixé de manière amovible au boîtier principal (1) au moyen d'un raccord.

2. Pompe de perfusion modulaire selon la revendication précédente, **caractérisée en ce que** la pompe de perfusion comprend un dispositif de commande (22-26) avec un élément utile (24) par l'intermédiaire duquel la source d'énergie (21) alimente le dispositif de circulation (8-11) en énergie et **en ce que** l'élément utile (24) est reçu par l'autre boîtier (20).

3. Pompe de perfusion modulaire selon l'une quelconque des revendications précédentes, **caractérisée en ce que** la pompe de perfusion comprend un dispositif de commende (22-26) doté d'un interrupteur (23) pour interrompre de manière commandée l'alimentation en énergie pour le dispositif de circulation (8-11) et **en ce que** l'interrupteur (23) est reçu par l'autre boîtier (20).

4. Pompe de perfusion modulaire selon l'une quelconque des deux revendications précédentes, **caractérisée en ce que** dans ou au niveau du boîtier de pompe (1, 2) se trouve un détecteur (12-14 ; 15) qui détecte une grandeur physique du dispositif de circulation (8-11) ou d'un élément conduisant le produit et émet un signal de sortie (D14 ; D15) représentant la grandeur détectée au niveau du dispositif de commande (22-26) de la pompe de perfusion, en fonction duquel le dispositif de commande (22-26) produit un signal de réglage (S8) pour le dispositif de circulation (8-11).

5. Pompe de perfusion modulaire selon la revendication précédente, **caractérisée en ce que** la source d'énergie (21) alimente le détecteur (12-14 ; 15) en énergie par le biais d'un interrupteur (25 ; 26) qui commande le dispositif de commande (22-26) au moyen d'un signal de réglage (S13 ; S15).

6. Pompe de perfusion modulaire selon l'une quelconque des deux revendications précédentes, **caractérisée en ce que** l'interrupteur (25 ; 26) est disposé dans l'autre boîtier (20).

7. Pompe de perfusion modulaire selon l'une quelconque des trois revendications précédentes, **caractérisée en ce que** le détecteur est un transmetteur de position et/ou de vitesse (12-14) qui détecte une position et/ou une vitesse du dispositif de circulation (8-11) et **en ce que** le signal de sortie (D14) du détecteur (12-14) représente la position et/ou la vitesse détectée.

8. Pompe de perfusion modulaire selon l'une quelconque des revendications 4 à 6, **caractérisée en ce que** le détecteur est un détecteur d'occlusion et/ou de fuite (15), qui détecte une occlusion et/ou une fuite dans un élément de la pompe à perfusion conduisant le produit et s'étendant du réservoir (3) jusqu'à une sortie d'une canule de perfusion (6).

9. Pompe de perfusion modulaire selon l'une quelconque des revendications 4 à 6, **caractérisée en ce que** le détecteur est un détecteur de débit qui est disposé directement devant une canule de perfusion (6) dans un élément conduisant le produit de la pompe de perfusion.

10. Pompe de perfusion modulaire selon l'une quelconque des revendications précédentes, **caractérisée en ce que** d'un système d'alimentation en énergie englobant la source d'énergie (21) pour le dispositif de circulation (8-11), seule une ou plusieurs lignes (27) pour la transmission d'énergie est ou sont disposée(s) dans ou au niveau du boîtier de pompe (1, 2).

11. Pompe de perfusion modulaire selon l'une quelconque des revendications précédentes, **caractérisée en ce que** d'un système d'alimentation en énergie englobant la source d'énergie (21) de la pompe de perfusion, seules des lignes (27, 28, 29) pour la transmission d'énergie sont disposées dans ou au niveau du boîtier de pompe (1, 2).

12. Pompe de perfusion modulaire selon l'une quelconque des revendications 2 à 11, **caractérisée en ce que** d'un système de commande et d'alimentation en énergie englobant la source d'énergie (21) et un dispositif de commande (22-26) pour le sens de circulation (8-11), seules des lignes (27, 28, 29, 31, 32) pour la transmission d'énergie et/ou la transmission de signal sont disposées dans ou au niveau du boîtier de pompe (1, 2).

13. Pompe de perfusion modulaire selon l'une quelconque des revendications précédentes, **caractérisée en ce que** le dispositif de circulation (8-11) comprend un moteur (8) et au moins un organe de circulation (11) entraîné par le moteur, qui agit sur le produit et le transporte ainsi, et **en ce que** la source d'énergie (21) alimente le moteur (8) avec l'énergie nécessaire.

14. Pompe de perfusion modulaire selon l'une quelconque des revendications précédentes, **caractérisée en ce que** le boîtier de pompe (1, 2) et l'autre boîtier (20) sont reliés ensemble au moyen d'au moins une ligne d'alimentation en énergie (27, 28, 29).

15. Pompe de perfusion modulaire selon la revendication précédente, **caractérisée en ce que** l'ensemble de la transmission d'énergie entre le boîtier de pompe (1, 2) et l'autre boîtier (20) s'effectue par lignes.

16. Pompe de perfusion modulaire selon l'une quelconque des revendications précédentes, **caractérisée en ce que** le boîtier de pompe (1, 2) et l'autre boîtier (20) sont reliés ensemble au moyen d'au moins une ligne de signal (31, 32).

17. Pompe de perfusion modulaire selon la revendication précédente, **caractérisée en ce que** l'ensemble de la transmission de signal entre le boîtier de pompe (1, 2) et l'autre boîtier (20) s'effectue par lignes.

18. Pompe de perfusion modulaire selon l'une quelconque des revendications précédentes, **caractérisée en ce que** la canule de perfusion (6) dépasse de la face inférieure du boîtier auxiliaire (2).

19. Pompe de perfusion modulaire selon l'une quelconque des revendications précédentes, **caractérisée en ce que** le boîtier auxiliaire (2) est fixé de manière mobile et guidé sur le boîtier principal (1).
